# EUROPEAN PATENT APPLICATION

(11) **EP 4 800 109 A1**
(43) Date of publication of application: **02.09.2026**
(21) Application number: 25798847.7
(22) Date of filing: 11.10.2025
(51) Int. Cl.: C12N 15/10, C12Q 1/6806, B01L 3/00

(54) **NUCLEIC ACID PROCESSING METHOD**

(30) Priority: 10.01.2025 CN 202510044048
(71) Applicant: HANGZHOU BIOER TECHNOLOGY CO., LTD., Hangzhou, Zhejiang 310000 (CN)
(72) Inventor: ZHAO, Yunpeng, Hangzhou, Zhejiang 310000 (CN); LI, Dong, Hangzhou, Zhejiang 310000 (CN); HE, Xianhan, Hangzhou, Zhejiang 310000 (CN)
(74) Representative: Monteiro Alves, Inês
(86) International application number: PCT/CN2025/126955
(87) International publication number: WO 2026/148949

(57) **Abstract**

Provided in the present invention is a nucleic acid processing method, relating to the technical field of biomolecule detection. The contents of the present nucleic acid processing method including: in the nucleic acid extraction process of the nucleic acid processing method, the magnetic beads can be stirred and dispersed in the liquid vortex; the magnetic beads can not only be suspended under the action of a magnetic field, but also avoid falling into other channels after moving away from the magnetic field, thereby reducing the loss of magnetic beads. The method can complete nucleic acid extraction while improving the accuracy of inspection, so as to achieve high-precision nucleic acid extraction, and moreover, the method can further realize high-precision nucleic acid amplification.

## Description

### CROSS-REFERENCE TO RELATED APPLICATION

The present invention claims priority to Chinese Patent Application No. 202510044048.0 filed with the China National Intellectual Property Administration on January 10, 2025, and entitled "NUCLEIC ACID PROCESSING METHOD", the contents of which are incorporated herein by reference in entirety.

### TECHNICAL FIELD

The present invention relates to the technical field of biomolecule detection, and specifically, to a nucleic acid processing method.

### BACKGROUND ART

Microfluidic chip, also referred to as a lab-on-a-chip, is a technology for manipulating fluids at the micrometer scale. This technology miniaturizes the fundamental functions of a chemical and biological laboratory onto a chip measuring only a few square centimeters.

Through interdisciplinary integration of analytical chemistry, micro-electro-mechanical processing, computer science, electronics, materials science, biology, medicine, and other disciplines, miniaturization, automation, integration and portability from sample processing to detection are achieved.

In the prior art, achieving high-precision nucleic acid extraction via microfluidic chips remains challenging.

### SUMMARY

A nucleic acid processing method is provided in the present invention, wherein the method can complete nucleic acid extraction, improve the accuracy of inspection, and achieve high-precision nucleic acid extraction.

Embodiments of the present invention can be implemented as follows.

A nucleic acid processing method is provided in the present invention, wherein the nucleic acid processing method includes:
S1: placing a microfluidic chip in an analyzer, wherein the microfluidic chip has an information extraction chamber, and a first liquid storage chamber, a second liquid storage chamber, a third liquid storage chamber and a fourth liquid storage chamber communicated with the information extraction chamber; a plurality of magnetic beads are accommodated in the information extraction chamber, and the first liquid storage chamber, the second liquid storage chamber, the third liquid storage chamber and the fourth liquid storage chamber respectively contain a lysis buffer, a first washing buffer, a second washing buffer and an elution buffer;
S2: adding a sample into the information extraction chamber;
S3: delivering the lysis buffer from the first liquid storage chamber to the information extraction chamber;
S4: controlling the microfluidic chip to rotate at a first preset speed for a first preset duration;
S5: controlling a magnetic element (magnetic steel) in the analyzer to be close to the microfluidic chip to suspend the magnetic beads in the information extraction chamber; herein, the method of step S5 further includes:
   S501: controlling the microfluidic chip to rotate for a third preset duration in a manner of accelerating rotation in a forward direction to a fourth preset speed and then decelerating;
   S502: controlling the microfluidic chip to rotate for the third preset duration in a manner of accelerating rotation in a reverse direction to the fourth preset speed and then decelerating; and
   S503: repeating steps S501-S502 to make the liquid in the information extraction chamber generate a vortex;
S6: controlling the rotation speed of the microfluidic chip to operate at a constant fifth preset speed to make the magnetic beads gather toward the side wall in the information extraction chamber;
S7: controlling the magnetic element (magnetic steel) to rise and move away from the microfluidic chip to stop the magnetic beads from suspending;
S8: heating a temperature-controlled air cavity to make the air in the temperature-controlled air cavity expand and enter the information extraction chamber, thus discharging the gas in the temperature-controlled air cavity; herein, the microfluidic chip further has the temperature-controlled air cavity, and the temperature-controlled air cavity is communicated with the information extraction chamber;
S9: cooling the temperature-controlled air cavity to make the temperature-controlled air cavity in a negative pressure state, thereby aspirating the liquid in the information extraction chamber into the temperature-controlled air cavity, such that the magnetic beads and the nucleic acids caught by the magnetic beads remain in the information extraction chamber;
S10: releasing the first washing buffer from the second liquid storage chamber into the information extraction chamber, and repeating steps S4-S9;
S11: releasing the second washing buffer from the third liquid storage chamber into the information extraction chamber, and repeating steps S4-S9; and
S12: releasing the elution buffer from the fourth liquid storage chamber into the information extraction chamber, and repeating steps S4-S7 to release the nucleic acids from the magnetic beads.

In an optional embodiment, after step S12, the nucleic acid processing method further includes:
S13: heating a temperature-controlled chamber to make the air in the temperature-controlled chamber expand and enter the information extraction chamber, so as to discharge the gas in the temperature-controlled chamber; herein, the microfluidic chip further has a detection chamber and the temperature-controlled chamber, and the information extraction chamber, the detection chamber, and the temperature-controlled chamber are sequentially communicated;
S14: cooling the temperature-controlled chamber to make the temperature-controlled chamber in a negative pressure state, so as to deliver the elution buffer with nucleic acids in the information extraction chamber to the detection chamber; and
S15: heating the detection chamber under a preset temperature condition to realize nucleic acid amplification.

In an optional embodiment, step S14 includes:
S141: cooling the temperature-controlled chamber to make the temperature-controlled chamber in a negative pressure state; and
S142: controlling the microfluidic chip to rotate at a constant speed greater than a second preset speed, whereby the liquid in the detection chamber enters the detection pool of the detection chamber under centrifugal action; or
controlling the microfluidic chip to rotate at a third preset speed for a second preset duration, then controlling the microfluidic chip to rotate at the second preset speed for the second preset duration, and then controlling the chip to rotate at the third preset speed for the second preset duration, thereby controlling in such a cycle to make the liquid in the detection chamber enter the detection pool of the detection chamber under centrifugal action, wherein the third preset speed is less than the second preset speed.

In an optional embodiment, the second preset duration is 0-10s, the second preset speed is 600rpm-6000rpm, and the third preset speed is 0-1000rpm.

In an optional embodiment, the preset temperature condition is 60°C-95°C.

In an optional embodiment, the first preset speed is 100rpm-6000rpm, and the first preset duration is 1s-700s.

In an optional embodiment, the diameter of the magnetic beads is 200nm-5000nm, and the fourth preset speed is 200rpm-3000rpm.

In an optional embodiment, the method of step S5 further includes:
controlling the information extraction chamber to heat up to above 37°C.

In an optional embodiment, the diameter of the magnetic beads is 100nm-5000nm.

In an optional embodiment, the fifth preset speed is 100rpm-900rpm.

The nucleic acid processing method in the embodiment of the present invention includes, for example, the following beneficial effects.

In the embodiment of the present invention, a nucleic acid processing method is provided, and the nucleic acid processing method includes: S1: placing a microfluidic chip in an analyzer; S2: adding a sample into an information extraction chamber; S3: delivering a lysis buffer in a first liquid storage chamber to the information extraction chamber; S4: controlling the microfluidic chip to rotate at a first preset speed for a first preset duration; S5: controlling a magnetic element (magnetic steel) in the analyzer to be close to the microfluidic chip to suspend magnetic beads in the information extraction chamber; S6: controlling the rotation speed of the microfluidic chip to operate at a constant fifth preset speed to gather the magnetic beads toward the side wall in the information extraction chamber; S7: controlling the magnetic element (magnetic steel) to rise and move away from the microfluidic chip to stop the magnetic beads from suspending; S8: heating a temperature-controlled air cavity to expand air in the temperature-controlled air cavity and make it enter the information extraction chamber, so as to discharge gas in the temperature-controlled air cavity, wherein the microfluidic chip further has the temperature-controlled air cavity, and the temperature-controlled air cavity is communicated with the information extraction chamber; S9: cooling the temperature-controlled air cavity to make the temperature-controlled air cavity in a negative pressure state, so as to aspirate liquid in the information extraction chamber into the temperature-controlled air cavity and leave the magnetic beads in the information extraction chamber; S10: releasing a first washing buffer from a second liquid storage chamber into the information extraction chamber, and repeating steps S4-S9; S11: releasing a second washing buffer from a third liquid storage chamber into the information extraction chamber, and repeating steps S4-S9; S12: releasing an elution buffer from a fourth liquid storage chamber into the information extraction chamber, and repeating steps S4-S7 to release nucleic acids from the magnetic beads. Herein, the method of step S5 further includes: S501: controlling the microfluidic chip to rotate for a third preset duration in a manner of accelerating rotation in a forward direction to a fourth preset speed and then decelerating; S502: controlling the microfluidic chip to rotate for a third preset duration in a manner of accelerating rotation in a reverse direction to the fourth preset speed and then decelerating; S503: repeating steps S501-S502 to generate a vortex in the liquid in the information extraction chamber. Through the present nucleic acid processing method, in the process of nucleic acid extraction, the magnetic beads can be stirred, thereby dispersing them in the liquid vortex; the magnetic beads can not only be suspended under the action of a magnetic field but also avoid falling into other channels after moving away from the magnetic field, thereby reducing the loss of magnetic beads. The nucleic acid processing method can complete nucleic acid extraction while improving the accuracy of inspection, so as to achieve high-precision nucleic acid extraction.

### BRIEF DESCRIPTION OF THE DRAWINGS

To more clearly illustrate the technical solutions of the embodiments of the present invention, the accompanying drawings required for the embodiments are briefly introduced below. It should be understood that the following accompanying drawings only show some embodiments of the present invention and should therefore not be regarded as limiting the scope. For those ordinarily skilled in the art, other relevant accompanying drawings can also be obtained based on these accompanying drawings without making inventive efforts.

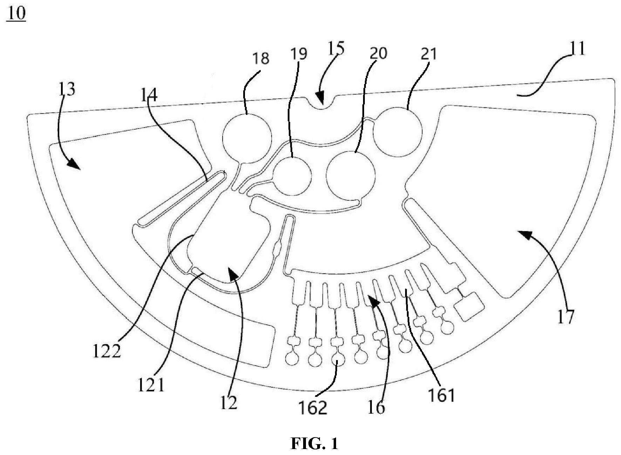

FIG. 1 is a schematic diagram of a microfluidic chip provided in an embodiment of the present invention.

Reference numerals: 10 - microfluidic chip; 11 - chip body; 12 - information extraction chamber; 121 - bottom wall; 122 - side wall; 13 - temperature-controlled air cavity; 14 - siphon tube; 15 - rotation center; 16 - detection chamber; 161 - quantitative pool; 162 - detection pool; 17 - temperature-controlled chamber; 18 - first liquid storage chamber; 19 - second liquid storage chamber; 20 - third liquid storage chamber; 21 - fourth liquid storage chamber.

### DETAILED DESCRIPTION OF EMBODIMENTS

To make the objectives, technical solutions and advantages of the embodiments of the present invention clearer, the technical solutions in the embodiments of the present invention are clearly and completely described below in conjunction with the accompanying drawings in the embodiments of the present invention. Obviously, the described embodiments are part of the embodiments of the present invention, rather than all the embodiments. Generally, the components of the embodiments of the present invention described and shown in the accompanying drawings herein can be arranged and designed in various different configurations.

Therefore, the following detailed description of the embodiments of the present invention provided in the accompanying drawings is not intended to limit the scope of the claimed present invention, but merely represents selected embodiments of the present invention. Based on the embodiments in the present invention, all other embodiments obtained by those ordinarily skilled in the art without making inventive efforts shall fall within the scope of protection of the present invention.

It should be noted that similar reference numerals and letters denote similar items in the following accompanying drawings; therefore, once an item is defined in one accompanying drawing, it need not be further defined or explained in subsequent accompanying drawings.

In the description of the present invention, it should be noted that if terms such as "upper", "lower", "inner" and "outer" appear, the orientations or positional relationships indicated are based on the orientations or positional relationships shown in the accompanying drawings, or the orientations or positional relationships in which the product of the present invention is usually placed when in use. These terms are only for the convenience of describing the present invention and simplifying the description, rather than indicating or implying that the referred device or element must have a specific orientation, be constructed and operated in a specific orientation. Therefore, these terms cannot be understood as limiting the present invention.

In addition, if terms such as "first" and "second" appear, they are only used for distinguishing and describing, and cannot be understood as indicating or implying relative importance.

It should be noted that, in the case of no conflict, the features in the embodiments of the present invention can be combined with each other.

The microfluidic chip has the characteristics of flexible combination and large-scale integration of various unit technologies, so that a large amount of information can be obtained with a small amount of samples, and it is more likely to exceed a single analysis function and become an integral micro-multivariate operation platform. Due to the advantages of small size, low reagent consumption, fast analysis speed, and easy integration, the microfluidic chip provides broad prospects for its application in many fields such as biomedical research, drug synthesis and screening, environmental monitoring, and sanitary quarantine.

As mentioned in the background art, the microfluidic chip, also referred to as a lab-on-a-chip, is a technology for manipulating fluids on a micrometer scale. This technology miniaturizes the fundamental functions of a chemical and biological laboratory onto a chip with a size of only a few square centimeters. Through interdisciplinary integration of analytical chemistry, micro-electro-mechanical processing, computer science, electronics, materials science, biology, medicine and other disciplines, miniaturization, automation, integration and portability from sample processing to detection are achieved.

In the prior art, it is difficult to realize high-precision nucleic acid extraction and amplification through a microfluidic chip. Specifically, although a microfluidic chip is provided in Patent CN116371491A, there is no appropriate control method to realize high-precision nucleic acid extraction and amplification.

In view of this, referring to FIG. 1, the nucleic acid processing method provided in the embodiment of the present invention can solve this problem, and it is described in detail below.

A nucleic acid processing method is provided in the embodiment of the present invention, wherein the nucleic acid processing method includes the following steps.
S1: A microfluidic chip 10 is placed in an analyzer, wherein the microfluidic chip is provided with an information extraction chamber 12, and a first liquid storage chamber 18, a second liquid storage chamber 19, a third liquid storage chamber 20 and a fourth liquid storage chamber 21 which are communicated with the information extraction chamber 12; a plurality of magnetic beads (the diameter of the magnetic beads can be 100nm-5000nm) are accommodated in the information extraction chamber; and the first liquid storage chamber 18, the second liquid storage chamber 19, the third liquid storage chamber 20 and the fourth liquid storage chamber 21 contain a lysis buffer, a first washing buffer, a second washing buffer and an elution buffer respectively.

It should be noted that, in the present embodiment, an existing microfluidic chip in the prior art can be selected as the microfluidic chip. The microfluidic chip is provided with a chip body 11, wherein an information extraction chamber 12, a first liquid storage chamber 18, a second liquid storage chamber 19, a third liquid storage chamber 20 and a fourth liquid storage chamber 21 are arranged on the chip body; the chip body is further provided with a rotation center 15, and the chip body 11 can rotate around the rotation center 15.

Herein, the microfluidic chip is further provided with a temperature-controlled air cavity 13 arranged on the chip body 11, and the temperature-controlled air cavity and the information extraction chamber 12 are communicated through a siphon tube 14.

S2: A sample is added into the information extraction chamber. Specifically, the information extraction chamber is communicated with a sample adding hole, wherein the sample is dropped or injected through the sample adding hole.

S3: The lysis buffer from the first liquid storage chamber 18 is delivered to the information extraction chamber; for example, the lysis buffer is delivered into the information extraction chamber 12 in an extrusion manner.

S4: The microfluidic chip is controlled to rotate in a forward direction or a reverse direction at a first preset speed (for example, the first preset speed is 100rpm-6000rpm) for a first preset duration (for example, the first preset duration is 1s-700s), so that both the lysis solution and the sample enter the bottom wall 121 of the information extraction chamber 12; and the forward rotation herein can be understood as the clockwise rotation of the microfluidic chip in FIG. 1, and the reverse rotation can be understood as the counterclockwise rotation of the microfluidic chip.

S5: A magnetic element (for example, magnetic steel) in the analyzer is controlled to be close to the microfluidic chip to suspend magnetic beads in the information extraction chamber. The process of the magnetic steel being close to the microfluidic chip herein can be understood as that the position of the microfluidic chip remains unchanged, the magnetic steel descends in height, and the magnetic field generated by the magnetic steel acts on the magnetic beads to make the magnetic beads suspend.

It should be noted that, in step S5, trehalose is mixed on the outer side of the magnetic beads, and the mixed state is in a dried state; under the action of the magnetic field, the magnetic beads can be quickly detached from the trehalose, so that the magnetic bead can quickly catch the nucleic acids.

Herein, the method of step S5 further includes the following steps.

S501: The microfluidic chip is controlled to rotate for a third preset duration (such as 1s) in a manner of accelerating rotation in a forward direction to a fourth preset speed (such as 600rpm) and then decelerating.

S502: The microfluidic chip is controlled to rotate for the third preset duration in a manner of accelerating rotation in a reverse direction to the fourth preset speed and then decelerating.

S503: Steps S501-S502 are repeated to generate a vortex in the liquid in the information extraction chamber.

Since the position of the magnetic steel remains unchanged, during the rotation of the microfluidic chip, the distance between the magnetic beads and the magnetic steel changes, and therefore, the magnetic beads move up and down; meanwhile, the rotation mode of the microfluidic chip continuously accelerates and decelerates while alternating between forward and reverse directions, inducing a vortex in the liquid, and finally, the magnetic beads are dispersed within the vortex.

A vortex is generated in the liquid in the information extraction chamber, so that the magnetic beads can be stirred and dispersed in the liquid vortex; in this way, the magnetic beads can not only be suspended under the action of the magnetic field but also avoid falling into other channels (the other channels herein can be understood as chambers communicated with the information extraction chamber) after moving away from the magnetic field, thereby reducing the loss of magnetic beads. It can complete nucleic acid extraction while improving the accuracy of inspection, so as to achieve high-precision nucleic acid extraction.

It should be noted that in some embodiments, when the diameter of the magnetic beads is 200nm-5000nm, the fourth preset speed is 200rpm-3000rpm.

In addition, the method of step S5 further includes: controlling the information extraction chamber to heat up to above 37°C. It can be understood that during the stirring of the magnetic beads, by controlling the information extraction chamber to heat up to above 37°C, the lysis efficiency can be improved.

S6: The rotation speed of the microfluidic chip is controlled to operate at a constant fifth preset speed; the fifth preset speed is 100rpm-900rpm, for example, the fifth preset speed can be 100rpm, 500rpm or 900rpm, so that the magnetic beads gather toward two opposite side walls 122 in the information extraction chamber. Generally, the rotation direction of the microfluidic chip is different, making the side wall 122 where the magnetic beads gather different; in this way, the magnetic beads can be prevented from falling into the channel opening through which the information extraction chamber communicates with the temperature-controlled air cavity, thus avoiding the loss of magnetic beads.

S7: The magnetic element is controlled to rise and move away from the microfluidic chip to stop the magnetic beads from suspending. Specifically, the magnetic element can be made to rise by controlling the magnetic field, for example, the distance between the magnetic element and the microfluidic chip is 5mm; after the magnetic beads stop suspending, they fall and gather at the bottom of the information extraction chamber (the bottom herein is understood as the bottom of the information extraction chamber along the direction of the rotation axis of the microfluidic chip).

S8: The temperature-controlled air cavity is heated to expand the air in the temperature-controlled air cavity and make it enter the information extraction chamber, so as to discharge the gas in the temperature-controlled air cavity.

S9: The temperature-controlled air cavity is cooled to make the temperature-controlled air cavity in a negative pressure state, so as to aspirate the liquid in the information extraction chamber into the temperature-controlled air cavity, and thus the magnetic beads and the nucleic acids caught by the magnetic beads are retained in the information extraction chamber.

S10: The first washing buffer from the second liquid storage chamber 19 is released into the information extraction chamber, and steps S4-S9 are repeated.

S11: The second washing buffer from the third liquid storage chamber 20 is released into the information extraction chamber, and steps S4-S9 are repeated.

S12: The elution buffer in the fourth liquid storage chamber 21 is released into the information extraction chamber, and steps S4-S7 are repeated to detach the nucleic acids from the magnetic beads.

To amplify nucleic acids, after step S12, the nucleic acid processing method further includes the following steps.

S13: The temperature-controlled chamber 17 is heated to expand the air in the temperature-controlled chamber and make it enter the information extraction chamber, so as to discharge the gas in the temperature-controlled chamber. Herein, the microfluidic chip is further provided with a detection chamber 16 arranged on the chip body and the temperature-controlled chamber 17 arranged on the chip body. It should be noted that the detection chamber 16 includes a quantitative pool 161 and a detection pool 162 which are communicated with each other, and the information extraction chamber, the quantitative pool 161 of the detection chamber 16 and the temperature-controlled chamber are communicated in sequence.

After the liquid (for example, the elution buffer with nucleic acids) enters the detection chamber 16, the quantitative pool 161 functions to quantitatively divide the liquid into equal parts, which is then delivered to the detection pool 162, ensuring the detection pool 162 is precisely filled without introducing air bubbles. S14: The temperature-controlled chamber is cooled to make the temperature-controlled chamber 17 in a negative pressure state, so as to deliver the elution buffer with nucleic acids in the information extraction chamber 12 to the detection chamber 16, while the magnetic beads are left in the information extraction chamber 12. Specifically, when the elution buffer with nucleic acids passes through the detection chamber 16, since the microfluidic chip is driven to rotate, the elution buffer with nucleic acids can be dispersed into different channels (the different channels herein can be understood as different quantitative pools 161 of the detection chamber 16 and a channel chamber of the detection chamber adjacent to the temperature-controlled chamber 17) of the detection chamber by the centrifugal force, and the excess elution buffer enters the temperature-controlled chamber for storage. It can be seen that the temperature-controlled chamber 17 functions as a pump and a waste liquid storage.

It should be noted that, as shown in FIG. 1, the volume of the channel chamber of one detection chamber adjacent to the temperature-controlled chamber 17 is larger than that of other channels; meanwhile, the channel chamber adjacent to the temperature-controlled chamber can function as a waste liquid pool, and the side wall interface through which the temperature-controlled chamber communicates with the channel chamber is closer to the rotation center.

In other embodiments, it can further be that, the channel chamber can be integrated with the temperature-controlled chamber, and the side wall interface through which the temperature-controlled chamber communicates with the channel chamber can be arranged at the middle position of the side wall of the temperature-controlled chamber.

S15: The detection chamber 16 is heated under a preset temperature condition (for example, the temperature of the preset temperature condition is 60°C-95°C), so as to realize nucleic acid amplification.

It should be noted that, in the present embodiment, the preset temperature condition is a series of temperature cycles; for example, the temperature can first reach 60°C and last for 5s, then the temperature is heated to 72°C and maintained for 10s, then the temperature is heated to 95°C and maintained for 15s, and then the temperature is cooled back to 60°C, and the cycle is performed in this manner.

In the present embodiment, step S14 includes the following steps.

S141: The temperature-controlled chamber is cooled, so as to make the temperature-controlled chamber in a negative pressure state.

S142: When the flow channel (the flow channel can be understood as the communication channel between the quantitative pool 161 and the detection pool 162) is relatively large, for example, when its size is larger than 500µm × 500µm, the microfluidic chip is controlled to rotate at a constant speed greater than the second preset speed, so that the liquid in the detection chamber enters the detection pool 162 of the detection chamber through the quantitative pool 161 under the action of centrifugal force; or
when the flow channel is relatively small, for example, when its size is smaller than 200µm × 200µm, the microfluidic chip is controlled to rotate at the third preset speed for a second preset duration, then the microfluidic chip is controlled to rotate at the second preset speed for the second preset duration, and then the microfluidic chip is controlled to rotate at the third preset speed for the second preset duration again; the control is performed in this cyclic manner, so as to make the liquid in the detection chamber enter the detection pool 162 of the detection chamber through the quantitative pool 161 under the action of centrifugal force, wherein the third preset speed is less than the second preset speed.

Herein, the second preset duration is 0-10s, the second preset speed is 600rpm-6000rpm, and the third preset speed is 0-1000rpm.

To sum up, the nucleic acid processing method can adopt nano-scale magnetic beads, by using the magnetic beads for stirring and separating multiple samples, and by means of the control method of the microfluidic chip, so as to realize high-precision nucleic acid extraction and amplification.

The above are only specific embodiments of the present invention, the protection scope of the present invention is not limited thereto. Any change or replacement that can be easily thought of by those skilled in the art within the technical scope disclosed by the present invention shall be covered within the protection scope of the present invention. Therefore, the protection scope of the present invention shall be subject to the protection scope of the claims.

### INDUSTRIAL APPLICABILITY

In the nucleic acid extraction process of the nucleic acid processing method provided by the present invention, the magnetic beads can be stirred and dispersed in the liquid vortex; the magnetic beads can not only be suspended under the action of a magnetic field, but also avoid falling into other channels after moving away from the magnetic field, thereby reducing the loss of magnetic beads. The method can complete nucleic acid extraction while improving the accuracy of inspection, so as to achieve high-precision nucleic acid extraction, and moreover, the method can further realize high-precision nucleic acid amplification.

## Claims

1. A nucleic acid processing method, **characterized in that** the nucleic acid processing method comprises:
S1: placing a microfluidic chip in an analyzer, wherein the microfluidic chip has an information extraction chamber, and a first liquid storage chamber, a second liquid storage chamber, a third liquid storage chamber and a fourth liquid storage chamber communicated with the information extraction chamber; a plurality of magnetic beads are accommodated in the information extraction chamber, and the first liquid storage chamber, the second liquid storage chamber, the third liquid storage chamber and the fourth liquid storage chamber respectively contain a lysis buffer, a first washing buffer, a second washing buffer and an elution buffer;
S2: adding a sample into the information extraction chamber;
S3: release the lysis buffer from the first liquid storage chamber to the information extraction chamber;
S4: controlling the microfluidic chip to rotate at a first preset speed for a first preset duration;
S5: controlling a magnetic element in the analyzer to be close to the microfluidic chip to suspend the magnetic beads in the information extraction chamber, wherein the method of step S5 further comprises:
S501: controlling the microfluidic chip to rotate for a third preset duration in a manner of accelerating rotation in a forward direction to a fourth preset speed and then decelerating;
S502: controlling the microfluidic chip to rotate for the third preset duration in a manner of accelerating rotation in a reverse direction to the fourth preset speed and then decelerating; and
S503: repeating steps S501-S502 to make a liquid in the information extraction chamber generate a vortex;
S6: controlling the rotation speed of the microfluidic chip to operate at a constant fifth preset speed to make the magnetic beads gather toward a side wall in the information extraction chamber;
S7: controlling the magnetic element to rise and move away from the microfluidic chip to stop the magnetic beads from suspending;
S8: heating a temperature-controlled air cavity to make the air in the temperature-controlled air cavity expand and enter the information extraction chamber, so as to discharge the gas in the temperature-controlled air cavity, wherein the microfluidic chip further has the temperature-controlled air cavity, and the temperature-controlled air cavity is communicated with the information extraction chamber;
S9: cooling the temperature-controlled air cavity to make the temperature-controlled air cavity in a negative pressure state, thereby aspirating the liquid in the information extraction chamber into the temperature-controlled air cavity, such that the magnetic beads and nucleic acids adsorbed by the magnetic beads remain in the information extraction chamber;
S10: releasing the first washing buffer from the second liquid storage chamber into the information extraction chamber, and repeating steps S4-S9;
S11: releasing the second washing buffer from the third liquid storage chamber into the information extraction chamber, and repeating steps S4-S9; and
S12: releasing the elution buffer from the fourth liquid storage chamber into the information extraction chamber, and repeating steps S4-S7 to detach the nucleic acids from the magnetic beads.

2. The nucleic acid processing method according to claim 1, wherein after step S12, the nucleic acid processing method further comprises:
S13: heating a temperature-controlled chamber to make the air in the temperature-controlled chamber expand and enter the information extraction chamber, so as to discharge the gas in the temperature-controlled chamber, wherein the microfluidic chip further has a detection chamber and the temperature-controlled chamber, and the information extraction chamber, the detection chamber and the temperature-controlled chamber are sequentially communicated;
S14: cooling the temperature-controlled chamber to make the temperature-controlled chamber in the negative pressure state, so as to deliver the elution buffer with the nucleic acids in the information extraction chamber to the detection chamber; and
S15: heating the detection chamber under a preset temperature condition to realize nucleic acid amplification.

3. The nucleic acid processing method according to claim 2, wherein step S14 comprises:
S141: cooling the temperature-controlled chamber to make the temperature-controlled chamber in the negative pressure state; and
S142: controlling the microfluidic chip to rotate at a constant speed greater than a second preset speed, whereby a liquid in the detection chamber enters a detection pool of the detection chamber under centrifugal action; or
controlling the microfluidic chip to rotate at a third preset speed for a second preset duration, then controlling the microfluidic chip to rotate at the second preset speed for the second preset duration, and then controlling the chip to rotate at the third preset speed for the second preset duration, thereby controlling in such a cycle to make a liquid in the detection chamber enter a detection pool of the detection chamber under centrifugal action, wherein the third preset speed is less than the second preset speed.

4. The nucleic acid processing method according to claim 3, wherein the second preset duration is 0-10s, the second preset speed is 600rpm-6000rpm, and the third preset speed is 0-1000rpm.

5. The nucleic acid processing method according to claim 2, wherein the preset temperature condition is 60°C-95°C.

6. The nucleic acid processing method according to claim 1, wherein the first preset speed is 100rpm-6000rpm, and the first preset duration is 1s-700s.

7. The nucleic acid processing method according to claim 1, wherein a diameter of the magnetic beads is 200nm-5000nm, and the fourth preset speed is 200rpm-3000rpm.

8. The nucleic acid processing method according to claim 1, wherein the method of step S5 further comprises:
controlling the information extraction chamber to heat up to above 37°C.

9. The nucleic acid processing method according to claim 1, wherein a diameter of the magnetic beads is 100nm-5000nm.

10. The nucleic acid processing method according to claim 1, wherein the fifth preset speed is 100rpm-900rpm.
